# FASCICULE DE BREVET EUROPEEN

(11) **EP 2 829 240 B1**
(45) Date de publication et mention de la délivrance du brevet: **12.12.2018**
(21) Numéro de dépôt: 14178466.0
(22) Date de dépôt: 25.07.2014
(51) Int. Cl.: A61B 17/29, A61F 2/24, A61B 17/04

(54) **Dispositif chirurgical**
Chirurgische Vorrichtung
Surgical device

(30) Priorité: 26.07.2013 FR 1357400
(43) Date de publication de la demande: 28.01.2015
(73) Titulaire: Landanger, 52906 Chaumont Cedex 9 (FR)
(72) Inventeur: Landanger, Benoit, 21490 Ruffey les Echirey (FR)
(74) Mandataire: Oudin, Stéphane

(56) Documents cités:
- WO-A1-2012/096280
- WO-A1-2013/008817
- US-A1- 2005 251 177
- US-A1- 2007 049 952
- US-A1- 2009 105 751
- US-A1- 2010 113 873
- US-B1- 7 635 386

## Description

### Domaine technique

La présente invention concerne notamment un dispositif chirurgical qui peut être notamment utilisé pour la pose de prothèse de cordage mitrale. Par ailleurs, la présente invention concerne également un procédé pour la pose d'une prothèse de cordage mitrale utilisant le dispositif selon l'invention.

La valve mitrale est une soupape qui sépare l'atrium gauche du ventricule gauche. Elle est plus particulièrement constituée d'un anneau fibreux, de deux feuillets, reliés au muscle du ventricule gauche par des piliers musculaires, par l'intermédiaire de cordages fibreux.

L'ouverture ou la fermeture de la valve mitrale est déclenchée par la différence de pression entre l'atrium gauche et le ventricule gauche. Lors de la phase de dilatation du ventricule, la valve est ouverte permettant le passage du sang de l'atrium vers le ventricule. Au début de la contraction de ventricule, la pression y remonte soudain, la valve se ferme et empêche le reflux.

L'insuffisance mitrale est une pathologie fréquente des valves cardiaques causée par une mauvaise fermeture des deux feuillets de celles-ci, entrainant un reflux de sang du ventricule gauche dans l'atrium gauche pendant la phase d'expulsion du sang. Les formes sévères d'insuffisance mitrale peuvent rendre nécessaire une opération chirurgicale.

Les principales causes de l'insuffisance mitrale sont la maladie coronarienne, les cardiomyopathies, ou le prolapsus mitral. Elle peut également apparaitre lors d'autres malformations congénitales du coeur.

Le traitement dépend de l'importance de la fuite, de son retentissement sur le ventricule gauche et du terrain.

Le traitement chirurgical peut consister soit en une reconstruction valvulaire (plastie mitrale), soit en un remplacement de la valve mitrale par une prothèse (valve artificielle) : valve mécanique ou bioprothèse valvulaire.

Dans le cadre d'une plastie mitrale, le chirurgien inspecte de façon minutieuse les dégâts de la valve mitrale. En fonction de la nature de la dégradation de la valve mitrale, le chirurgien procède à une couture de cette valve ou à une réparation sur les cordages de la valve. Un anneau est très souvent mis en place autour de la valve de manière à ce que les feuillets de la valve soient bien étanches. Parfois, lorsque la valve est perforée, le chirurgien réalise un patch (petite membrane) à l'aide d'un bout de péricarde (tissu qui entoure le coeur) qu'il prélève sur le malade lors de l'intervention.

Dans le cadre de cette intervention, il peut donc être nécessaire de remplacer le cordage mitrale par une prothèse prenant la forme d'un fil en goretex.

Il existe donc un besoin, pour un dispositif permettant d'introduire la prothèse au niveau du coeur et de l'insérer sur la valve mitrale.

US 2005/251177 A1 divulgue un dispositif chirurgical comprenant un cylindre creux, une mâchoire composée de deux mors et un tube creu où lesdits mors comprennent chacun un passage et où le tube creu est associé à un des mors de telle sorte que ledit tube creux débouche dans ledit passage et en ce que l'extrémité distale dudit tube creu est montée en rotation, autour d'un axe perpendiculaire à l'axe longitudinal dudit passage, entre une position haute où l'extrémité distale dudit tube creux est dans le prolongement dudit passage et une position basse où l'extrémité distale dudit tube creux est disposée perpendiculairement audit passage.WO 2012/096280 A1 divulgue un dispositif chirurgical comprenant les caractéristiques du préambule.

### Résumé de l'invention

La présente invention concerne notamment un dispositif chirurgical, comme revendiqué dans la première revendication, comprenant un cylindre creux, une mâchoire composé de deux mors, comprenant une surface extérieure et une surface interne, montés pivotant par rapport à un axe perpendiculaire à l'axe longitudinal dudit cylindre creux entre une position ouverte et une position fermée, où la mâchoire est disposée dans le prolongement de l'extrémité distale dudit cylindre creux et deux tubes creux, remarquable en ce que lesdits mors comprennent chacun un passage de ladite surface externe à ladite surface interne, lesdits passages étant disposés en vis-à-vis lorsque ladite mâchoire est en position fermée et en ce que chacun desdits tubes creux est associé respectivement à un des mors de telle sorte que ledit tube creux débouche dans ledit passage.

Selon un mode de réalisation préféré de l'invention, lesdits tubes creux sont souples.

Selon un mode de réalisation préféré de l'invention, lesdits tubes creux sont logés en partie à l'intérieur dudit cylindre creux.

Selon l'invention, l'extrémité distale desdits tubes creux est montée en rotation, autour d'un axe perpendiculaire à l'axe longitudinal dudit passage, entre une position haute où l'extrémité distale dudit tube creux est dans le prolongement dudit passage et une position basse où l'extrémité distale dudit tube creux est disposée perpendiculairement audit passage.

Selon un mode de réalisation encore plus préféré de l'invention, la surface externe desdits mors comprend à sa surface externe une rainure destinée à recevoir la partie distale dudit tube creux lorsque celui-ci est en position basse.

Selon un mode de réalisation préféré de l'invention, la rotation desdits mors est entrainée par une tige, placée en tout ou partie dans ledit cylindre creux, dont l'extrémité proximale débouche à l'extrémité proximale dudit cylindre creux et dont l'extrémité distale est associée à l'extrémité proximale desdits mors.

Selon un mode de réalisation préféré de l'invention, la surface interne desdits mors comporte des cannelures.

Selon un mode de réalisation encore plus préféré de l'invention, lesdites cannelures présentent en section droite un profil triangulaire.

Selon un mode de réalisation préféré de l'invention, ledit cylindre creux comporte en son extrémité distale une ouverture disposée dans le prolongement de ladite rainure et destinée à permettre le passage dudit tube creux de l'intérieur à l'extérieur dudit cylindre.

Selon un mode de réalisation préféré de l'invention, ledit cylindre creux comprend en sa partie proximale des ouvertures destinées à permettre le passage desdits tubes creux de l'intérieur à l'extérieur dudit cylindre creux.

Selon un mode de réalisation préféré de l'invention, l'extrémité distale de ladite mâchoire présente, en coupe longitudinale, un profil courbe.

Selon un mode de réalisation préféré de l'invention l'extrémité de ladite mâchoire présente, en coupe longitudinale, un profil hémicirculaire en position fermée.

Selon un mode de réalisation préféré de l'invention, ladite mâchoire est associée audit cylindre creux via une pièce intermédiaire comprenant une partie proximale, venant s'insérer dans l'extrémité distale dudit cylindre creux, et une partie distale comprenant des évidements aptes à recevoir l'axe guidant en rotation lesdits mors.

### Brèves description des dessins

La figure 1 présente une vue de dessus d'un mode de réalisation d'un dispositif selon l'invention.
La figure 2 présente une vue en coupe longitudinale du mode de réalisation de l'invention présentée à la figure 1.
La figure 3 présente une vue en perspective d'un dispositif selon l'invention avec les mâchoires en position ouvertes.

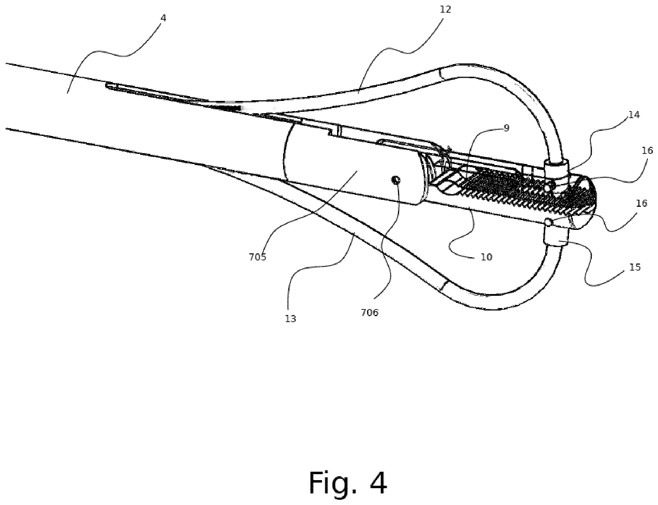

La figure 4 présente une vue en perspective d'un dispositif selon l'invention avec les mâchoires en position fermées.

### Description des modes de réalisation

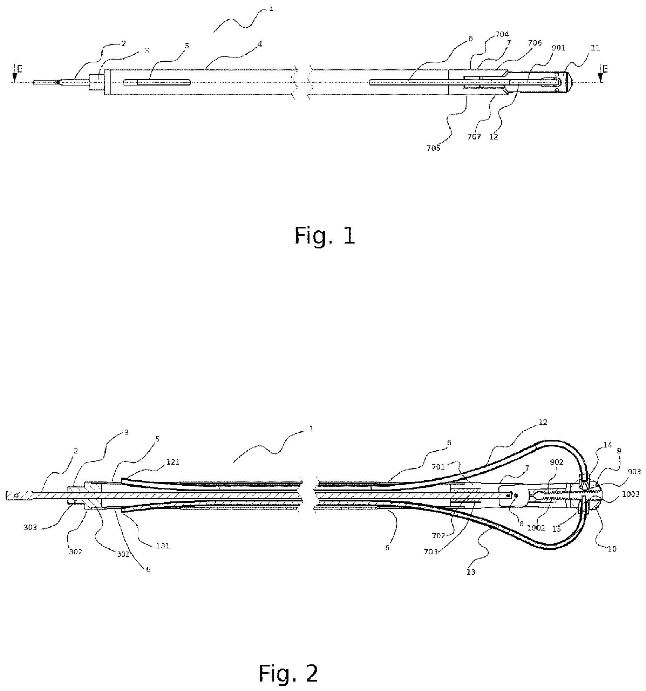

En référence aux figures 1 et 2, le dispositif 1 selon l'invention comporte de son extrémité proximale à son extrémité distale : l'extrémité libre de la tige 2, le bouchon d'obturation 3, le cylindre creux 4 comportant, sur sa face supérieure et sur sa face inférieure, une ouverture sous la forme d'un trou oblong 5 et une rainure 6 dans l'axe du trou oblong, la pièce intermédiaire 7 supportant l'axe 8 autour duquel les mors 9, 10 sont mis en rotation.

La rainure 6 se prolonge jusqu'à l'extrémité distale dudit cylindre creux puis au niveau de la pièce intermédiaire 7 puis sur la surface extérieure du mors adjacent 9. Cette rainure 6 et destinée à recevoir un tube creux 12, 13 lorsque celui-ci est en position basse. Ainsi, on comprend que lorsque que la mâchoire 11 est fermée, et les tubes creux 12, 13 en position basse, l'ensemble du dispositif 1 selon l'invention s'inscrit totalement dans un cylindre de diamètre égal à celui dudit cylindre creux.

En référence à la figure 2, le bouchon d'obturation 3 comporte préférentiellement une gorge 301 venant s'insérer à l'intérieur du cylindre et un épaulement 302 venant en appui sur l'extrémité proximale du cylindre. Ce bouchon d'obturation 3 comporte un alésage 303 permettant le passage de la tige 2. Le bouchon d'obturation 3 est préférentiellement constitué dans un matériau permettant d'assurer l'étanchéité au niveau de la jonction alésage/tige 2. Il permet ainsi l'obturation du cylindre creux 4 et le maintien de la tige 2 selon un axe parallèle à l'axe longitudinal du cylindre creux 4.

Le cylindre creux 4 a un diamètre préférentiellement compris entre 3 et 10 millimètres. Son extrémité distale est obturée par la pièce intermédiaire 7 dont la partie proximale forme un bouchon 701 venant s'insérer un l'intérieur du cylindre creux 4. La partie externe de ce bouchon 701 a un diamètre équivalent au diamètre du cylindre creux 4. Ce bouchon est alésé afin de permettre le passage de la tige 2, cette tige 2 est donc supportée à la fois par le bouchon d'obturation 3 à la partie proximale du cylindre creux 4 et par la pièce intermédiaire 7 située à la partie distale dudit cylindre creux 4. L'alésage 702 de ladite pièce intermédiaire 7 est préférentiellement recouvert d'un joint cylindrique 703.

La pièce intermédiaire 7 comprend en outre une deuxième partie constituée de deux lames 704, 705 s'étendant en direction de la mâchoire 11 et supportant l'axe 8 destiné à maintenir les mors 9, 10. L'axe 8 est maintenu, au niveau de ses extrémités, dans un trou percé 706, 707 dans chacune desdites lames 704, 705. L'espace présent entre les deux lames est situé dans le prolongement de la rainure 6, présente sur la première partie de la pièce intermédiaire 7.

La mâchoire 11 est donc constituée de deux mors 9, 10 maintenus en rotation autour de l'axe 8 entre une position ouverte et une position fermée. Le passage de la position ouverte à ladite position fermée est préférentiellement réalisé via la tige 2 dont l'extrémité distale est associée aux mors 9, 10 de la mâchoire 11 par une association de type biellette/rainure.

La surface externe desdits mors 9,10 comprend une rainure 901, 1001 placée dans le prolongement de l'espace entre les deux lames 704, 705 de la pièce intermédiaire 7. La surface interne desdits mors 9, 10 comprend des cannelures 902, 1002 permettant d'améliorer le maintien de la mâchoire 11 au niveau de la valvule lorsque ladite mâchoire 11 est en position fermée. Lesdites cannelures 902, 1002 présentent préférentiellement, en section droite, un profil triangulaire. Toutefois, on comprend bien que ledit profil peut être modifié en fonction des besoins.

Afin de faciliter la pénétration du dispositif 1 selon l'invention à l'intérieur du corps du patient à traiter, l'extrémité distale de la mâchoire 11 a préférentiellement une surface courbe et encore plus préférentiellement une surface hémisphérique.

Chacun des deux mors 9, 10 comprend une surface interne et une surface externe et un passage 903, 1003 de sa surface externe à sa surface interne disposée de telle sorte que lesdits passages 903, 1003 sont en vis-à-vis lorsque la mâchoire 11 est en position fermée.

Le dispositif 1 selon l'invention comprend par ailleurs deux tubes creux 12, 13 ouverts à chacune de leurs extrémités. Chacun desdits tubes creux 12, 13 est logé en partie à l'intérieur du cylindre creux 4 et comprend une extrémité libre 121,131, débouchant à l'extérieur du cylindre creux 4 via le trou oblong 5 placé à la partie proximale du cylindre creux 4, et une extrémité associée à un mors 9, 10 de la mâchoire 11. Afin d'assurer l'étanchéité du cylindre creux 4, ledit trou oblong 5 et/ou ladite rainure 6 sont obturés par un joint permettant le passage dudit tube creux 12, 13.

L'association entre l'extrémité du tube creux 12, 13 et du mors 9, 10 est faite de telle sorte que l'ouverture du tube 12, 13 à cette extrémité soit à l'aplomb du passage 903, 1003 dudit mors, lorsque ledit tube creux 9, 10 est en position haute, de telle sorte que ledit tube creux 9, 10 débouche dans ledit passage 903, 1003.

Avantageusement, l'extrémité distale des tubes creux 12, 13 est montée en rotation, autour d'un axe perpendiculaire à l'axe longitudinal dudit passage 903, 1003, entre une position haute où l'extrémité distale dudit tube creux 12, 13 est dans le prolongement dudit passage 903, 1003 et une position basse ou l'extrémité distale dudit tube creux 12, 13 est disposée perpendiculairement audit passage 903, 1003. Préférentiellement, cette association est réalisée via une tête cylindrique 14, 15 comportant un alésage dans lequel vient s'insérer l'extrémité distale dudit tube creux 12, 13. Cette tête cylindrique comprend deux ergots 16, 17, disposés co-axialement de part et d'autre de sa surface externe, venant s'insérer dans deux logements congruents 18, 19 disposés à l'intérieur dudit passage 903, 1003. On comprend ainsi que la tête cylindrique 14, 15 va pouvoir pivoter, autour de l'axe défini par les ergots 16, 17, entre une position haute et une position basse.

L'association entre l'extrémité distale du tube creux 12, 13 et l'alésage de la tête cylindrique 14, 15 est préférentiellement réalisée par collage. Préférentiellement, une des deux têtes cylindriques, dite tête d'émission 15, est associée à l'extrémité distale du tube creux 13 sur toute la longueur de son alésage. L'extrémité distale du tube creux 13 venant ainsi affleurer au niveau de l'extrémité distale de la tête cylindrique 15. L'autre tête cylindrique, dite tête réceptrice 16, comprend, de son extrémité proximale vers son extrémité distale un alésage prolongé par une fraisure s'évasant vers l'extrémité distale de ladite tête réceptrice 16. Dans ce cas, l'extrémité distale du tube creux 12 est associée à l'alésage de ladite tête réceptrice 16 et vient affleurer au niveau de l'entrée de la fraisure.

L'extrémité distale desdites têtes cylindriques 14, 15 présente préférentiellement une surface hémisphérique.

En position basse, l'extrémité distale dudit tube creux 12, 13 est placée perpendiculairement à l'axe longitudinal du passage 9003, 1003 reliant la surface interne à la surface externe du mors 9,10. Le tube creux 12, 13 est ainsi logé entièrement à l'intérieur de la rainure 901, 1001 présente à la surface externe dudit mors puis dans l'espace entre les deux lames de la pièce intermédiaire 7 puis dans la rainure 6 à la surface de la première partie de ladite pièce intermédiaire 7. Dans cette position, ledit tube creux 12, 13 est donc intégralement logé à l'intérieur des pièces constituant le reste du dispositif 1 selon l'invention.

En position haute, l'extrémité distale dudit tube creux 12, 13 est dans le prolongement dudit passage 903, 1003 reliant la surface externe à la surface interne dudit mors 9, 10. Le passage de la position haute à la position basse se fait en poussant sur l'extrémité libre 121, 131 dudit tube 12, 13 qui va entraîner la rotation de la tête cylindrique 14,15 autour de ses ergots 16, 17. Le passage de la position haute à la position basse se fait par un mouvement inverse.

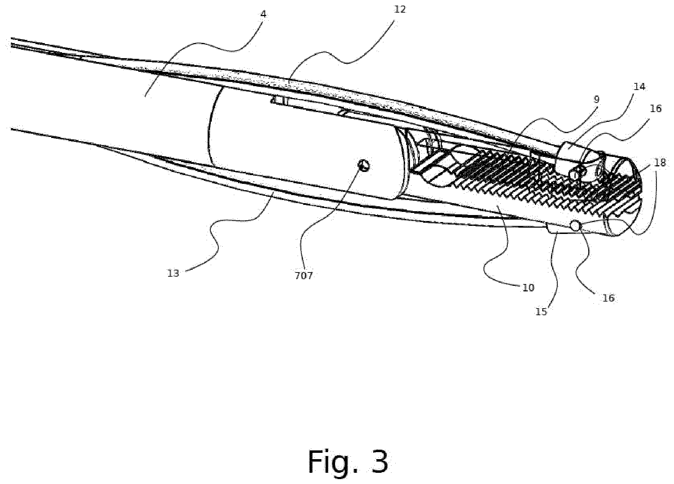

Le dispositif 1 selon l'invention fonctionne de la manière suivante :
1. Le praticien fait entrer la mâchoire 11 dans le coeur du patient avec les tubes creux 12, 13 en position basse (voir figure 3).
2. Il ouvre les mors 9,10 pour saisir la valve mitrale.
3. Il verrouille la mâchoire 11 en position fermée sur la mitrale.
4. Il pousse sur les tubes creux 12, 13 pour les mettre en position haute.
5. Il fait ensuite passer une tige fine flexible à laquelle est attachée la prothèse de cordage en goretex dans les éléments suivants dans l'ordre :
   a. Le tube creux 13 associé à la tête cylindrique d'émission 15
   b. La tête cylindrique d'émission 15
   c. Le premier mors 10
   d. La valve mitrale : on la traverse (le bout de la tige fine flexible servant d'aiguille)
   e. Le second mors 9
   f. La tête cylindrique de réception 14
   g. Le tube creux associé à la tête cylindrique de réception 12
6. On tire ensuite sur la tige pour positionner le cordage synthétique et retirer la tige-aiguille.

## Revendications

1. Dispositif (1) chirurgical comprenant un cylindre creux (4), une mâchoire (11) composée de deux mors (9, 10), comprenant une surface extérieure et une surface interne, montés pivotant par rapport à un axe (8) perpendiculaire à l'axe longitudinal dudit cylindre creux (4) entre une position ouverte et une position fermée, où la mâchoire (11) est disposée dans le prolongement de l'extrémité distale dudit cylindre creux (4) et deux tubes creux (12, 13), où lesdits mors (9, 10) comprennent chacun un passage (903, 1003) de ladite surface externe à ladite surface interne, lesdits passages (903, 1003) étant disposés en vis-à-vis lorsque ladite mâchoire (11) est en position fermée, **caractérisé en ce que** chacun desdits tubes creux (12, 13) est associé respectivement à un des mors (9, 10) de telle sorte que ledit tube creux (12, 13) débouche dans ledit passage (903, 1003) et **en ce que** l'extrémité distale desdits tubes creux (12,13) est montée en rotation, autour d'un axe perpendiculaire à l'axe longitudinal dudit passage (903, 1003), entre une position haute où l'extrémité distale dudit tube creux (12, 13) est dans le prolongement dudit passage (903, 1003) et une position basse où l'extrémité distale dudit tube creux (12, 13) est disposée perpendiculairement audit passage (903, 1003).

2. Dispositif (1) chirurgical selon la revendication précédente **caractérisé en ce que** la surface externe desdits mors (9, 10) comprend à sa surface externe une rainure (901, 1001) destinée à recevoir la partie distale dudit tube creux (12, 13) lorsque celui-ci est en position basse.

3. Dispositif (1) chirurgical selon l'une des revendications précédentes **caractérisé en ce que** la rotation desdits mors (9, 10) est entrainée par une tige (2), placée en tout ou partie dans ledit cylindre creux (4), dont l'extrémité proximale débouche à l'extrémité proximale dudit cylindre creux (4) et dont l'extrémité distale est associée à l'extrémité proximale desdits mors (9, 10).

4. Dispositif (1) chirurgical selon l'une des revendications précédentes **caractérisé en ce que** la surface interne desdits mors (9, 10) comporte des cannelures.

5. Dispositif (1) chirurgical selon la revendication précédente **caractérisé en ce que** lesdites cannelures présentent en section droite un profil triangulaire.

6. Dispositif (1) chirurgical selon l'une des revendications précédentes **caractérisé en ce que** ledit cylindre creux (4) comporte en son extrémité distale des ouvertures disposées dans le prolongement desdites rainures (901, 1001) et destinée à permettre le passage dudit tube creux (12, 13) de l'intérieur à l'extérieur dudit cylindre creux (4).

7. Dispositif (1) chirurgical selon l'une des revendications précédentes **caractérisé en ce que** ledit cylindre creux (4) comprend en sa partie proximale des ouvertures (5) destinées à permettre le passage desdits tubes creux (12, 13) de l'intérieur à l'extérieur dudit cylindre creux (4).

8. Dispositif (1) chirurgical selon l'une des revendications précédentes **caractérisé en ce que** ladite mâchoire 11 est associée audit cylindre creux (4) via une pièce intermédiaire (7) comprenant une partie proximale, venant s'insérer dans l'extrémité distale dudit cylindre creux (4), et une partie distale comprenant des évidements aptes à recevoir l'axe guidant (8) en rotation lesdits mors (9, 10).

9. Dispositif (1) chirurgical selon l'une des revendications précédentes **caractérisé en ce que** lorsque lesdits tube creux (12, 13) sont en position basse et que la mâchoire (11) est fermée, l'ensemble du dispositif (1) selon l'invention s'inscrit totalement dans un cylindre de diamètre égal à celui dudit cylindre creux (4).

## Patentansprüche

1. Chirurgische Vorrichtung (1), umfassend einen hohlen Zylinder (4), eine Klaue (11) bestehend aus zwei Klemmbacken (9, 10), eine äußere Oberfläche und eine innere Oberfläche umfassend, die im Verhältnis zu einer zur Längsachse des hohlen Zylinders (4) senkrechten Achse (8) zwischen einer offenen Position und einer geschlossenen Position schwenkbar montiert sind, wobei die Klaue (11) in der Verlängerung des distalen Endes des hohlen Zylinders (4) angeordnet ist, und zwei hohle Rohre (12, 13), wobei die Klemmbacken (9, 10) jeweils einen Durchlass (903, 1003) von der äußeren Oberfläche zu der inneren Oberfläche umfassen, wobei die Durchlässe (903, 1003) einander gegenüber angeordnet sind, wenn die Klaue (11) in geschlossener Position ist, **dadurch gekennzeichnet, dass** jedes der hohlen Rohre (12, 13) jeweils einer der Klemmbacken (9, 10) zugeordnet ist, sodass das hohle Rohr (12, 13) in den Durchlass (903, 1003) mündet, und dadurch, dass das distale Ende der hohlen Rohre (12, 13) um eine zur Längsachse des Durchlasses (903, 1003) senkrechte Achse zwischen einer oberen Position, in der das distale Ende des hohlen Rohres (12, 13) in der Verlängerung des Durchlasses (903, 1003) ist, und einer unteren Position drehend montiert ist, in der das distale Ende des hohlen Rohres (12, 13) senkrecht zu dem Durchlass (903, 1003) angeordnet ist.

2. Chirurgische Vorrichtung (1) nach dem vorstehenden Anspruch, **dadurch gekennzeichnet, dass** die äußere Oberfläche der Klemmbacken (9, 10) an ihrer äußeren Oberfläche eine Nut (901, 1001) umfasst, die dazu bestimmt ist, den distalen Teil des hohlen Rohres (12, 13) aufzunehmen, wenn dieses in der unteren Position ist.

3. Chirurgische Vorrichtung (1) nach einem der vorstehenden Ansprüche, **dadurch gekennzeichnet, dass** die Drehung der Klemmbacken (9, 10) durch einen Stift (2) angetrieben wird, der vollkommen oder teilweise in dem hohlen Zylinder (4) platziert ist, dessen proximales Ende in das proximale Ende des hohlen Zylinders (4) mündet und dessen distales Ende dem proximalen Ende der Klemmbacken (9, 10) zugeordnet ist.

4. Chirurgische Vorrichtung (1) nach einem der vorstehenden Ansprüche, **dadurch gekennzeichnet, dass** die innere Oberfläche der Klemmbacken (9, 10) Riffelungen beinhaltet.

5. Chirurgische Vorrichtung (1) nach dem vorstehenden Anspruch, **dadurch gekennzeichnet, dass** die Riffelungen im geraden Querschnitt ein Dreiecksprofil aufweisen.

6. Chirurgische Vorrichtung (1) nach einem der vorstehenden Ansprüche, **dadurch gekennzeichnet, dass** der hohle Zylinder (4) an seinem distalen Ende Öffnungen beinhaltet, die in der Verlängerung der Nuten (901, 1001) angeordnet sind und die dazu bestimmt sind, den Durchlass des hohlen Rohres (12, 13) von innerhalb nach außerhalb des hohlen Zylinders (4) zu ermöglichen.

7. Chirurgische Vorrichtung (1) nach einem der vorstehenden Ansprüche, **dadurch gekennzeichnet, dass** der hohle Zylinder (4) in seinem proximalen Teil Öffnungen (5) umfasst, die dazu bestimmt sind, den Durchlass der hohlen Rohre (12, 13) von innerhalb nach außerhalb des hohlen Zylinders (4) zu ermöglichen.

8. Chirurgische Vorrichtung (1) nach einem der vorstehenden Ansprüche, **dadurch gekennzeichnet, dass** die Klaue 11 dem hohlen Zylinder (4) über ein Zwischenstück (7) zugeordnet ist, das einen proximalen Teil umfasst, der in das distale Ende des hohlen Zylinders (4) eingeführt wird, und einen distalen Teil, der Ausnehmungen umfasst, die imstande sind, die die Klemmbacken (9, 10) drehführende Achse (8) aufzunehmen.

9. Chirurgische Vorrichtung (1) nach einem der vorstehenden Ansprüche, **dadurch gekennzeichnet, dass** wenn die hohlen Rohre (12, 13) in der unteren Position sind und die Klaue (11) geschlossen ist, die gesamte erfindungsgemäße Vorrichtung (1) vollständig in einem Zylinder enthalten ist, dessen Durchmesser gleich jenem des hohlen Zylinders (4) ist.

## Claims

1. Surgical device (1) comprising a hollow cylinder (4), a clamp (11) consisting of two jaws (9, 10), comprising an outer surface and an inner surface, pivotably mounted relative to an axis (8) perpendicular to the longitudinal axis of said hollow cylinder (4) between an open position and a closed position, wherein the clamp (11) is arranged extending from the distal end of said hollow cylinder (4) and two hollow tubes (12, 13), wherein said jaws (9, 10) each comprise a passage (903, 1003) from said outer surface to said inner surface, said passages (903, 1003) being arranged facing each other when said clamp (11) is in the closed position, **characterized in that** each of said hollow tubes (12, 13) is associated respectively with one of the jaws (9, 10) in such a way that said hollow tube (12, 13) opens into said passage (903, 1003) and **in that** the distal end of said hollow tubes (12,13) is rotatably mounted, about an axis perpendicular to the longitudinal axis of said passage (903, 1003), between a high position wherein the distal end of said hollow tube (12, 13) extends from said passage (903, 1003) and a low position wherein the distal end of said hollow tube (12, 13) is arranged perpendicularly to said passage (903, 1003).

2. Surgical device (1) according to the above claim **characterized in that** the outer surface of said jaws (9, 10) comprises at the outer surface thereof a groove (901, 1001) intended to receive the distal part of said hollow tube (12, 13) when said tube is in the low position.

3. Surgical device (1) according to any of the above claims **characterized in that** the rotation of said jaws (9, 10) is actuated by a rod (2), positioned wholly or partially in said hollow cylinder (4), wherein the proximal end opens at the proximal end of said hollow cylinder (4) and wherein the distal end is associated with the proximal end of said jaws (9, 10).

4. Surgical device (1) according to any of the above claims **characterized in that** the inner surface of said jaws (9, 10) comprises ribs.

5. Surgical device (1) according to the above claim **characterized in that** said ribs have a triangular transverse profile.

6. Surgical device (1) according to any of the above claims **characterized in that** said hollow cylinder (4) comprises at the distal end thereof openings arranged extending from said grooves (901, 1001) and intended to enable the passage of said hollow tube (12, 13) from inside to outside said hollow cylinder (4).

7. Surgical device (1) according to any of the above claims **characterized in that** said hollow cylinder (4) comprises in the proximal part thereof openings (5) intended to enable the passage of said hollow tubes (12, 13) from inside to outside said hollow cylinder (4).

8. Surgical device (1) according to any of the above claims **characterized in that** said clamp 11 is associated with said hollow cylinder (4) via an intermediate part (7) comprising a proximal part, inserted into the distal end of said hollow cylinder (4), and a distal part comprising recesses suitable for receiving the axis (8) guiding said jaws (9, 10) in rotation.

9. Surgical device (1) according to any of the above claims **characterized in that** when said hollow tubes (12, 13) are in the low position and said clamp (11) is closed, the whole device (1) according to the invention fits completely into a cylinder having a diameter equal to that of said hollow cylinder (4).
